# EUROPEAN PATENT APPLICATION

(11) **EP 4 772 105 A1**
(43) Date of publication of application: **08.07.2026**
(21) Application number: 24859067.1
(22) Date of filing: 24.05.2024
(51) Int. Cl.: A61B 3/024

(54) **VISUAL FIELD TESTING DEVICE, VISUAL FIELD TESTING METHOD, AND PROGRAM**

(30) Priority: 31.08.2023 JP 2023141403
(71) Applicant: QD Laser, Inc., Kawasaki-shi, Kanagawa 210-0855 (JP)
(72) Inventor: SUZUKI, Makoto, Kawasaki-shi, Kanagawa 210-0855 (JP)
(74) Representative: Becker Kurig & Partner Patentanwälte mbB
(86) International application number: PCT/JP2024/019288
(87) International publication number: WO 2025/047018

(57) **Abstract**

Provided is a visual field testing device that enables measurement of retinal sensitivity without increasing the burden on a subject. The present invention is provided with: a light irradiation unit that irradiates the retina of a subject with stimulation light; a projection control unit that causes the light irradiation unit to perform irradiation with the stimulation light with a testing pattern in which the intensity of the stimulation light is increased to a predetermined luminance over time; a switch that is operated by the subject having visually recognized the stimulation light; and a calculation unit that calculates the intensity of the stimulation light that can be visually recognized by the subject on the basis of the timing at which the switch is operated in the testing pattern.

## Description

### Technical Field

The disclosure relates to a visual field testing device, a visual field testing method, and a program.

### Related Art

Conventionally, a visual field testing device has been known that presents a fixation point in a housing covering a visual field of a subject, randomly displays a luminous point (optotype) within the visual field of the subject while the subject directs a line of sight toward the fixation point, and tests whether the subject can visually recognize the luminous point. A technique for determining an intensity of the stimulation light to be used during testing based on a threshold measured in advance by a response of the subject is disclosed in Patent Document 1.

### Citation List

### Patent Literature

Patent Literature: Japanese Patent Application Laid-Open Publication No. H08-117186

### SUMMARY OF INVENTION

### Technical Problem

In a conventional visual field testing device, since an intensity of the stimulation light applied to each site on the retina is fixed, retinal sensitivity of each individual subject cannot be measured. Further, for example, it is conceivable to test whether the subject can visually recognize the stimulation light for each intensity while switching the intensity of the stimulation light. However, in this case, testing time becomes longer, and a burden on a subject becomes greater.

Thus, in one aspect, the present invention aims to provide a visual field testing device capable of measuring the retinal sensitivity without increasing a burden on a subject.

### Solution to Problem

In one aspect,
a visual field testing device is provided that includes a light irradiation portion that irradiates stimulation light to a retina of the subject,
a projection control portion, configured to cause the light irradiation portion to irradiate the stimulation light with a testing pattern that increases an intensity of the stimulation light to a predetermined luminance over time;
a switch, configured to be operated by the subject who visually recognizes the stimulation light; and
a calculation portion, configured to calculate the intensity of the stimulation light that is visually recognizable by the subject based on a timing at which the switch is operated in the testing pattern.

### Effects of Invention

In one aspect, according to the present invention, an absolute value of the retinal sensitivity may be measured without increasing a burden on a subject.

### BRIEF DESCRIPTION OF THE DRAWINGS

[FIG. 1] is a diagram showing a configuration of a visual field testing device of the present embodiment.
[FIG. 1A] is a diagram illustrating an appearance of a testing instrument to which the visual field testing device of the present embodiment is applied.
[FIG. 2] is a diagram showing a configuration of a light irradiation portion.
[FIG. 3] is a diagram showing a site where stimulation light (laser light) is presented on a retina.
[FIG. 4] is a flowchart showing a visual field testing process in the visual field testing device of the present embodiment.
[FIG. 5] is a flowchart showing a reaction time measurement process for measuring a reaction time of a subject.
[FIG. 6] is a flowchart showing a calculation process in a calculation portion.
[FIG. 7] is a diagram showing a configuration example of a testing pattern of stimulation light and a reaction time measurement pattern during execution of a visual field test.
[FIG. 8] is a diagram showing a count value of an irradiation duration acquired in the reaction time measurement process of FIG. 5.
[FIG. 9] is a diagram showing a method for calculating the retinal sensitivity of a predetermined site on a retina of a subject.
[FIG. 10] is a diagram showing an example of accelerating a timing of transitioning a testing target to a next site on a retina in response to an operation on a switch.

### DESCRIPTION OF THE EMBODIMENTS

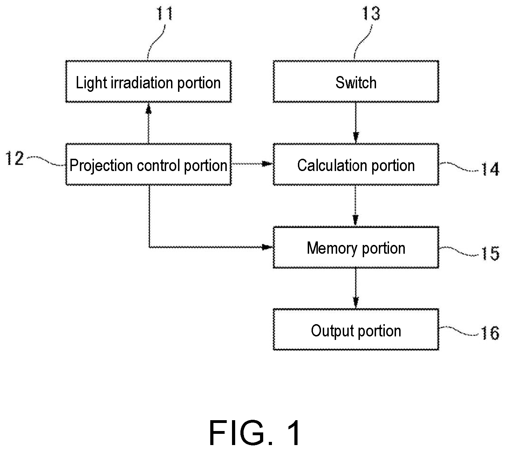

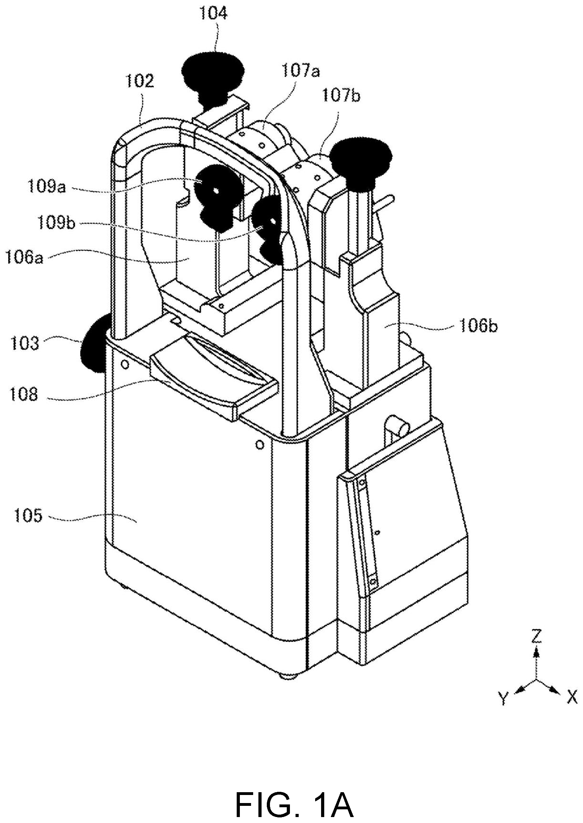

FIG. 1 is a diagram showing a configuration of a visual field testing device of the present embodiment.

As shown in FIG. 1, the visual field testing device of the present embodiment includes a light irradiation portion 11 that irradiates stimulation light onto a retina of a subject, a projection control portion 12 that causes the light irradiation portion 11 to irradiate the stimulation light with a testing pattern that increases an intensity of the stimulation light to a predetermined luminance over time, a switch 13 operated by the subject who visually recognizes the stimulation light, and a calculation portion 14 that calculates the intensity of the stimulation light that is visually recognizable by the subject based on a timing at which the switch 13 is operated in the testing pattern.

Further, the visual field testing device of the present embodiment includes a memory portion 15 that stores data related to testing and data such as testing results calculated by the calculation portion 14, and an output portion 16 that outputs the testing results and the like stored in the memory portion 15.

The visual field testing device of the present embodiment may be configured using a computer in which a predetermined program is implemented.

FIG. 1A is a diagram illustrating an appearance of a testing instrument to which the visual field testing device of the present embodiment is applied. In the example of FIG. 1A, the light irradiation portion 11 is housed inside the testing instrument. Although FIG. 1A shows a testing instrument corresponding to both left and right eyes, a testing instrument corresponding to testing of each of the left and right eyes individually may also be used. The configuration of the testing instrument is arbitrary, and for example, a testing instrument in which the light irradiation portion 11 is built into a device in the shape of goggles or eyeglasses that may be worn by a subject may also be used.

The testing instrument shown in FIG. 1A has a frame 102, adjustment members 103 and 104, a base portion 105, support portions 106a and 106b, image projection portions 107a and 107b, a pedestal 108, and imaging portions 109a and 109b. Further, the pedestal 108 is provided on the base portion 105, and two ends of the frame 102 are fixed to an upper surface of the base portion 105.

The image projection portions 107a and 107b are supported by the support portions 106a and 106b. The image projection portions 107a and 107b irradiate stimulation light to a left eye and a right eye of a subject whose vision such as visual field, visual acuity, and visual recognition is tested by the light irradiation portion 11 and project a projected image including a testing image onto the retina of the subject.

The adjustment member 103 moves the support portions 106a and 106b, on which the image projection portions 107a and 107b are supported, in the Y-axis direction shown in FIG. 1A. The adjustment member 104 moves the image projection portion 107 along the support portion 106 in the Z-axis direction shown in FIG. 1A.

In the visual testing device 100, testing is performed in a state where the image projection portion 107 is brought close to the eyeball such that a chin and a forehead of the subject are brought into contact with the pedestal 108 and the frame 102, respectively, and the subject is in a state of looking into the image projection portions 107a and 107b by the adjustment members 103 and 104.

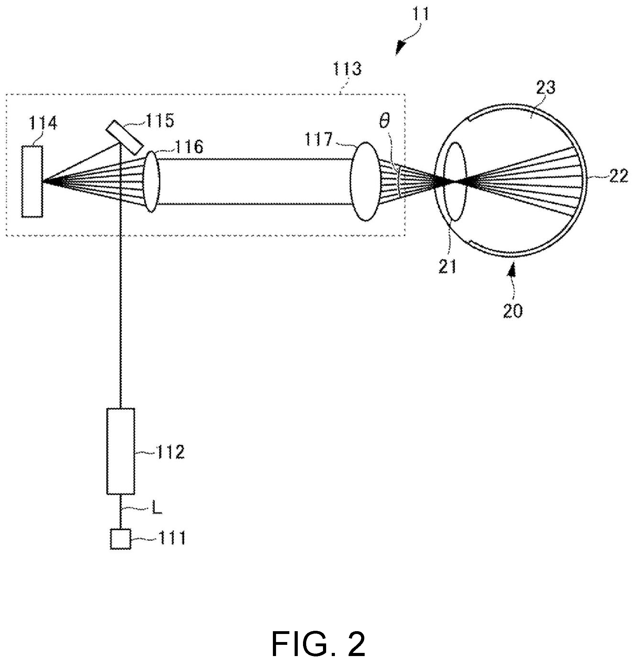

FIG. 2 is a diagram showing a configuration of the light irradiation portion. The light irradiation portion 11 irradiates stimulation light (laser light) onto the retina 22 of the eyeball 20 of the subject using Maxwellian view.

As shown in FIG. 2, the light irradiation portion 11 has a light source 111, an adjustment portion 112, and an optical system 113. The optical system 113 has a scanning portion 114, a plane mirror 115, and lenses 116 and 117. The scanning portion 114 is, for example, a 2-axis MEMS (Micro Electro Mechanical Systems) mirror.

In the light irradiation portion 11, the laser light L emitted from the light source 111 has a numerical aperture (NA) and/or a beam diameter adjusted in the adjustment portion 112. The laser light L is visible laser light such as red laser light, green laser light, and blue laser light.

The laser light L is reflected by the plane mirror 115 and scanned two-dimensionally by the scanning portion 114. The scanned laser light L is irradiated onto the eyeball 20 of the subject via the lens 116 and the lens 117. The laser light L converges near the crystalline lens 21, passes through the vitreous body 23, and is irradiated onto the retina 22. Thereby, an image is projected onto the retina 22. The scanning portion 114 vibrates at a relatively high frequency such as 28 kHz so that, for example, 60 frames of images are projected per second.

The position of the eyeball 20 relative to the light irradiation portion 11 is adjusted by, for example, the adjustment members 103 and 104 so that the laser light L is not vignetted (not blocked) at the position of the pupil of the eyeball 20. For example, by obtaining a positional relationship such that the laser light L diffused by scanning converges near the crystalline lens 21 of the eyeball 20 in the optical axis direction, it becomes possible to form an image in the entire visual field without the periphery of the image being lost.

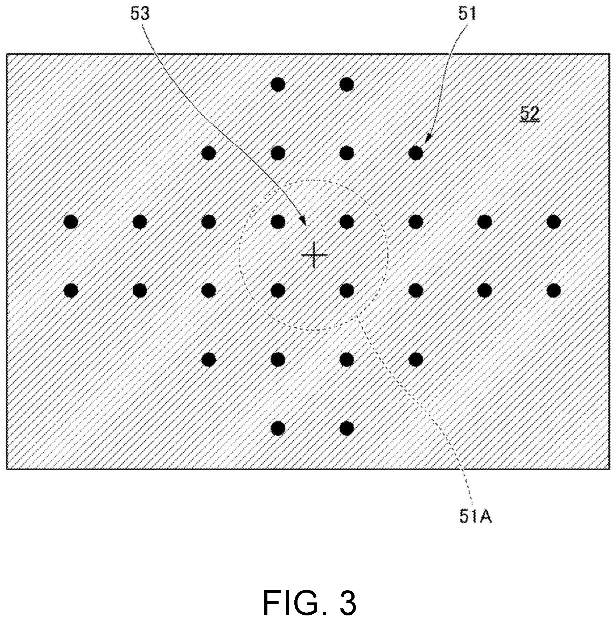

FIG. 3 is a diagram showing sites where stimulation light (laser light) is presented on the retina. In FIG. 3, each site is shown by a drawn dot 51.

As shown in FIG. 3, stimulation light is presented at a total of 28 sites on the retina 22. The interval between adjacent sites corresponds to an interval of 6 degrees as an angle of the stimulation light projected from the convergence point near the center of the crystalline lens 21 onto the retina 22, in both the vertical direction and the horizontal direction. A background 52 of predetermined luminance (gray color) is projected onto a region (rectangular region) including a region where the 28 sites are distributed. An arbitrary value may be selected as the luminance of the background 52, but in the following description, an example is shown in which the luminance of the background 52 is set to a brightness corresponding to a value of, for example, 100 out of a total of 256 gradations. Further, a mark 53 indicating a site where the subject performs central fixation is presented at the central portion of the region where the 28 sites are distributed. The subject performs a visual field test by operating the switch 13 when recognizing the stimulation light at the 28 sites while fixating on the mark 53.

Next, the operation of the visual field testing device of the present embodiment will be described.

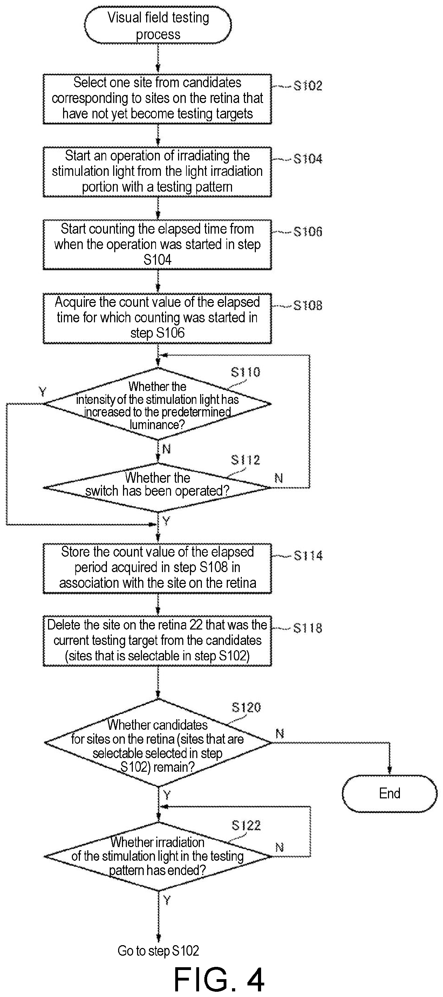

FIG. 4 is a flowchart showing a visual field testing process in the visual field testing device of the present embodiment.

In step S102 of FIG. 4, the projection control portion 12 selects one site from candidates corresponding to sites on the retina 22 that have not yet become testing targets.

In step S104, the projection control portion 12 starts an operation of irradiating the stimulation light from the light irradiation portion 11 onto the selected site on the retina 22 with a testing pattern that increases the intensity of the stimulation light to a predetermined luminance over time. For example, in the testing pattern, the light amount at the site is continuously increased by continuously increasing the 8-bit 256 gradation values from 100 corresponding to the luminance of the background 52 to 256. The time required to increase the light amount may be set, for example, in a range of 2 to 4 seconds.

In step S106, the projection control portion 12 starts counting the elapsed time from when the operation was started in step S104.

In step S108, the projection control portion 12 acquires the count value of the elapsed time for which counting was started in step S106.

In step S110, the projection control portion 12 determines, based on the count value of the elapsed time acquired in step S108, whether the intensity of the stimulation light has increased to the predetermined luminance described above, and if the determination is affirmative, the process proceeds to step S114, and if the determination is negative, the process proceeds to step S112.

In step S112, the projection control portion 12 determines whether the switch 13 has been operated, and if the determination is affirmative, the process proceeds to step S114, and if the determination is negative, the process proceeds to step S110.

In step S114, the memory portion 15 stores the count value of the elapsed period acquired in step S108 in association with the site on the retina 22.

In step S118, the projection control portion 12 deletes the site on the retina 22 that was the current testing target from the candidates (sites that is selectable in step S102).

In step S120, the projection control portion 12 determines whether candidates for sites on the retina 22 (sites that are selectable in step S102) remain, and if the determination is affirmative, the process proceeds to step S122, and if the determination is negative, the process ends.

In step S122, the projection control portion 12 determines, based on the count value of the elapsed time acquired in step S108, whether irradiation of the stimulation light in the testing pattern has ended, and if the determination is affirmative, the process proceeds to step S102, and if the determination is negative, the process of step S122 is repeated.

As described above, in the present embodiment, in the visual field testing process of FIG. 4, the count values of the elapsed period are acquired and stored for all 28 sites on the retina 22. Here, in the case where the count value of the elapsed period indicates an abnormality (error), the test (steps S104 to S114) may be executed again for the relevant site on the retina 22 to acquire a valid count value of the elapsed time.

In the present embodiment, the visual field testing process of FIG. 4 may be executed multiple times (for example, three times) to improve the accuracy of the testing result. In this case, the average value of the count values of the elapsed period acquired for each site on the retina 22 may be used as the final result (count value of the elapsed period) corresponding to the relevant site.

In the present embodiment, at a predetermined timing during execution of the process shown in FIG. 4, the stimulation light is irradiated from the light irradiation portion 11 in the reaction time measurement pattern, and the reaction time of the subject is measured.

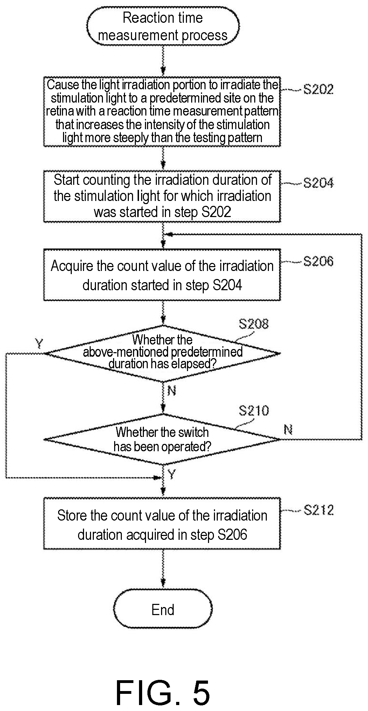

FIG. 5 is a flowchart showing a reaction time measurement process for measuring the reaction time of the subject. The reaction time measurement process is executed at random timing, for example, during execution of the process shown in FIG. 4.

In step S202 of FIG. 5, the projection control portion 12 causes the light irradiation portion 11 to irradiate the stimulation light to a predetermined site on the retina 22 with a reaction time measurement pattern that increases the intensity of the stimulation light more steeply than the testing pattern. The reaction time measurement pattern is, for example, a pattern in which the luminance of the stimulation light instantaneously rises from the luminance of the background 52 to a predetermined luminance (for example, a luminance of 256 as a gradation value) and continues for a predetermined duration. Further, as the site on the retina 22 to which the reaction time measurement pattern is applied, for example, a site in the central region 51A shown in FIG. 3 (in the example of FIG. 3, one of the four sites) is selected. This allows the reaction time of the subject to be measured more accurately.

In step S204, the projection control portion 12 starts counting the irradiation duration of the stimulation light for which irradiation was started in step S202.

In step S206, the projection control portion 12 acquires the count value of the irradiation duration started in step S204.

In step S208, the projection control portion 12 determines whether the above-mentioned predetermined duration has elapsed based on the count value of the duration acquired in step S108, and if the determination is affirmative, the process proceeds to step S212, and if the determination is negative, the process proceeds to step S210.

In step S210, the calculation portion 14 determines whether the switch 13 has been operated, and if the determination is affirmative, the process proceeds to step S212, and if the determination is negative, the process proceeds to step S206.

In step S212, the memory portion 15 stores the count value of the irradiation duration acquired in step S206, and the process ends.

In the present embodiment, the reaction time measurement process shown in FIG. 5 is performed multiple times while executing the process shown in FIG. 4, and multiple count values of the irradiation duration are acquired. This allows the reaction time of the subject to be accurately grasped. The execution count of the reaction time measurement process is arbitrary, but may be executed, for example, about 3 to 5 times. In the case where the count value of the irradiation duration acquired in step S206 indicates an abnormality (error), the execution count of the reaction time measurement process may be increased to secure a sufficient number of valid count values of the irradiation duration.

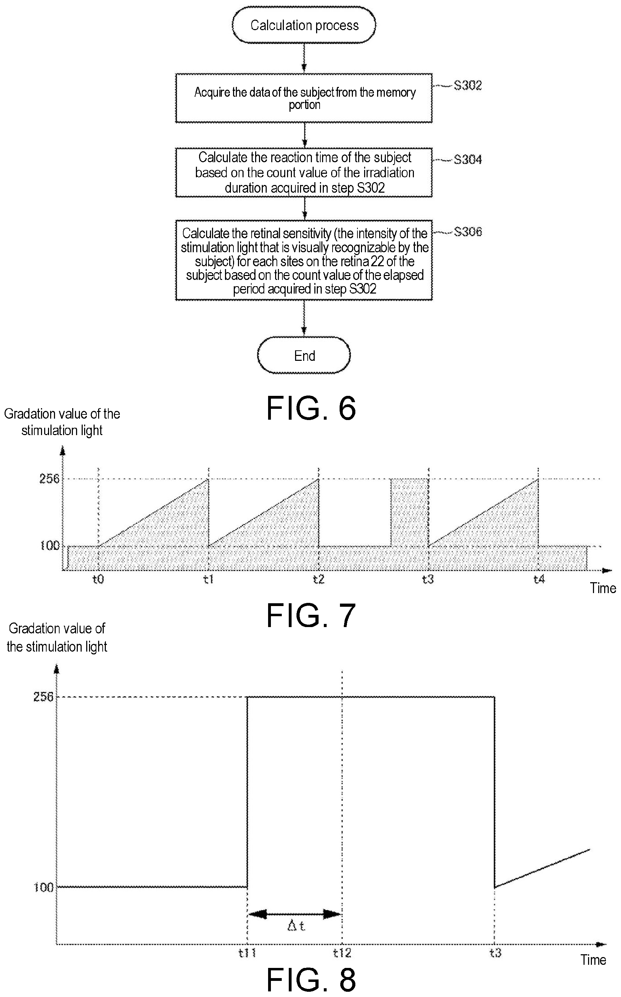

FIG. 6 is a flowchart showing the calculation process in the calculation portion.

In step S302 of FIG. 6, the calculation portion 14 acquires the data of the corresponding subject stored in the process of FIG. 4 from the memory portion 15. The data of the subject includes the count value of the irradiation duration (step S212) and the count value of the elapsed period (step S114).

In step S304, the calculation portion 14 calculates the reaction time of the subject based on the count value of the irradiation duration acquired in step S302. The reaction time may be calculated, for example, as an average value of the multiple count values of the irradiation duration. Further, in the case where the count values of the irradiation duration include values considered to be errors (values that are too small and values that are too large), the reaction time may be calculated as an average value of the remaining count values after excluding such count values.

In step S306, the calculation portion 14 calculates the retinal sensitivity (the intensity of the stimulation light that is visually recognizable by the subject) for each sites on the retina 22 of the subject based on the count value of the elapsed period acquired in step S302, and the process ends.

FIG. 7 is a diagram showing a configuration example of the testing pattern of the stimulation light and the reaction time measurement pattern during execution of the visual field test.

In the example of FIG. 7, basically, irradiation of the stimulation light by the testing pattern is sequentially repeated to sites on different retinas 22 at a constant period (for example, 2 to 4 seconds) (see step S122). However, within that period, the reaction time measurement process shown in FIG. 5 is inserted as an interrupt process (the interrupt process is not shown in FIG. 4). In FIG. 7, irradiation of the stimulation light by the testing pattern is started at times t0, t1, and t3, and irradiation of the stimulation light by the reaction time measurement pattern is performed between time t2 and time t3. In the testing pattern, the intensity continuously increases from a gradation value of 100 corresponding to the luminance of the background 52 (FIG. 3) to a gradation value of 256. Further, the intensity of the stimulation light by the reaction time measurement pattern corresponds to a gradation value of 256.

FIG. 8 is a diagram showing the count value of the irradiation duration acquired in the reaction time measurement process of FIG. 5.

In the example of FIG. 8, at time t11, the intensity of the stimulation light by the reaction time measurement pattern rises from a gradation value of 100 to 256, and at time t3, the intensity returns from a gradation value of 256 to 100. In the case where the switch 13 is operated at time t12 (in the case where the determination in step S210 is affirmative), the count value of the irradiation duration is acquired (stored) as a count value Δt.

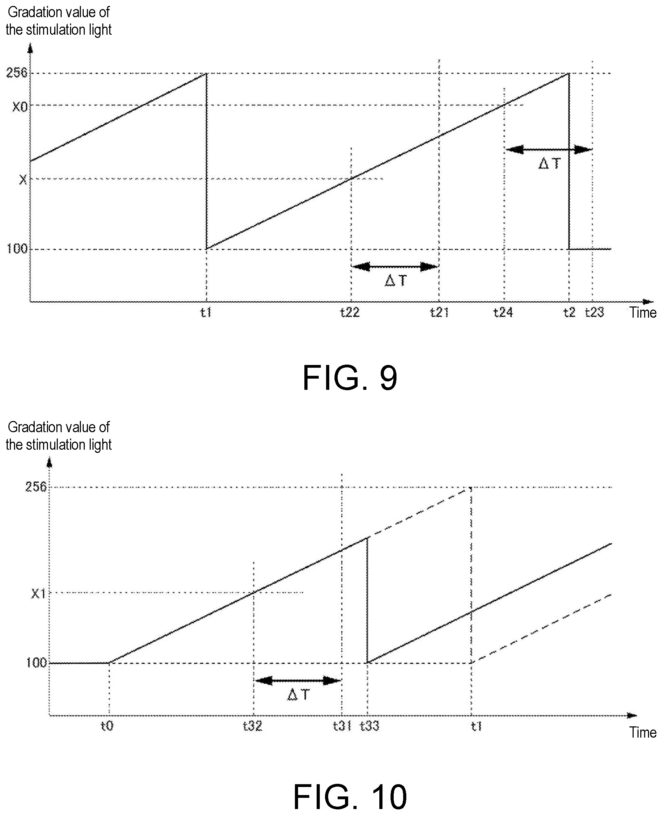

FIG. 9 is a diagram showing a method for calculating the retinal sensitivity of a predetermined site on the retina of the subject.

As described above, in the calculation process shown in FIG. 6, the reaction time of the subject is calculated based on the count value of the irradiation duration.

In the example of FIG. 9, the reaction time of the subject corresponding to the count value of the irradiation duration is shown as a reaction time ΔT.

In FIG. 9, the intensity of the stimulation light by the testing pattern gradually increases in gradation value from 100 starting from time t1, and reaches a gradation value of 256 at time t2. Here, in the case where the switch 13 is operated at time t21, the calculation portion 14 regards that the subject recognized the stimulation light at time t22, which is a time point earlier than time t21 by the reaction time ΔT. Thus, the calculation portion 14 calculates the gradation value X of the stimulation light corresponding to the time point of time t22 as the retinal sensitivity (the intensity of the stimulation light that is visually recognizable by the subject) of the corresponding site on the retina 22.

In some cases, the switch 13 may be operated after the intensity of the stimulation light by the testing pattern reaches a gradation value of 256, that is, after the time point (time t2) when the irradiation of the stimulation light by the testing pattern ends. In FIG. 9, in the case where the switch 13 is operated at time t23, the calculation portion 14 regards that the subject recognized the stimulation light at time t24, which is a time point earlier than time t23 by the reaction time ΔT. Thus, in this case, the gradation value X0 of the stimulation light corresponding to the time point of time t24 is calculated as the retinal sensitivity (the intensity of the stimulation light that is visually recognizable by the subject) of the corresponding site on the retina 22.

The retinal sensitivity of each site on the retina 22 calculated by the calculation portion 14 is stored in the memory portion 15, and the stored data is output by the output portion 16. For example, each site on the retina 22 may be shown as a two-dimensional chart as shown in FIG. 3, and may be displayed in a display mode corresponding to the retinal sensitivity of each site in the chart, that is, the intensity (gradation value) of the stimulation light that is visually recognizable by the subject. For example, each region in the chart may be displayed in a display color (for example, display colors with different gradations) corresponding to the retinal sensitivity of the corresponding site.

FIG. 10 is a diagram showing an example of accelerating the timing of transitioning the testing target to the next site on the retina in response to an operation on the switch.

In the case where there is an operation on the switch 13 corresponding to the selected site, the projection control portion 12 may transition the testing target to the next site on the retina 22 before increasing the luminance of the stimulation light by the testing pattern to the end.

In the example of FIG. 10, the stimulation light by the testing pattern is initially set such that its gradation value gradually increases from 100 starting from time t0 and reaches a gradation value of 256 at time t1. However, in the case where the subject's operation on the switch 13 occurs at time t31, it is regarded that the subject recognized the stimulation light at time t32, which is a time point earlier than time t31 by the reaction time ΔT, and the gradation value X1 of the stimulation light corresponding to the time point of time t32 is calculated as the retinal sensitivity (the intensity of the stimulation light that is visually recognizable by the subject) of the corresponding site on the retina 22. Then, in this case, after time t31 when the switch 13 is operated, the necessity of continuing the irradiation of the stimulation light by the testing pattern is lost. For this reason, in the example of FIG. 10, the irradiation of the stimulation light by the testing pattern is stopped at time t33, and the testing target is transitioned to the next site on the retina 22.

By adopting such an operation, in the case where the subject's operation on the switch 13 is performed early, the testing target may be promptly transitioned to the next site on the retina 22. For this reason, the testing time may be shorten and the burden on the subject may be reduced.

As described above, in the visual field testing device of the present embodiment, a testing pattern that increases the intensity of the stimulation light to a predetermined luminance over time is used, and the intensity of the stimulation light that is visually recognizable by the subject is calculated based on the timing at which the subject operates the switch. For this reason, the retinal sensitivity at each site on the retina 22 of each individual subject may be calculated without increasing the burden on the subject. Thus, for example, differentiation between diseases caused by opacity such as cataracts and diseases caused by visual field defects such as glaucoma becomes easy.

Further, even for a subject with low sensitivity, occurrence of a case where nothing is visible from the beginning to the end during the test may be prevented, and causing frustration and burden on the subject may be avoided.

Although each embodiment has been described in detail above, the disclosure is not limited to the specific embodiments, and various modifications and changes are possible within the scope described in the claims. Further, it is possible to combine all or multiple of the constituent elements of the above-described embodiments.

### Reference Signs List

- 11: Light irradiation portion
- 12: Projection control portion
- 13: Switch
- 14: Calculation portion
- 15: Memory portion
- 16: Output portion

## Claims

1. A visual field testing device comprising:
a light irradiation portion, configured to irradiate stimulation light onto a retina of a subject;
a projection control portion, configured to cause the light irradiation portion to irradiate the stimulation light with a testing pattern that increases an intensity of the stimulation light to a predetermined luminance over time;
a switch, configured to be operated by the subject who visually recognizes the stimulation light; and
a calculation portion, configured to calculate the intensity of the stimulation light that is visually recognizable by the subject based on a timing at which the switch is operated in the testing pattern.

2. The visual field testing device according to claim 1, wherein the projection control portion causes the light irradiation portion to irradiate the stimulation light with a reaction time measurement pattern that increases the intensity of the stimulation light more steeply than the testing pattern, and
the calculation portion calculates the intensity of the stimulation light that is visually recognizable by the subject based on a timing at which the switch is operated in the reaction time measurement pattern and a timing at which the switch is operated in the testing pattern.

3. The visual field testing device according to claim 2, wherein the projection control portion selects in a random order one of a plurality of sites on the retina and causes the light irradiation portion to irradiate the stimulation light with the testing pattern onto the site selected.

4. The visual field testing device according to claim 3, wherein the projection control portion interrupts with the reaction time measurement pattern at a random timing during testing with the testing pattern.

5. The visual field testing device according to claim 3, wherein in response to an operation of the switch corresponding to the site selected, the projection control portion shifts a testing target to a next site selected from a plurality of sites on the retina before increasing the intensity of the stimulation light to the predetermined luminance.

6. A visual field testing method comprising:
a projection control step of irradiating stimulation light with a testing pattern that increases an intensity of the stimulation light to a predetermined luminance over time; and
a calculation step of calculating the intensity of the stimulation light that is visually recognizable by a subject based on a timing at which a switch operated by the subject who visually recognizes the stimulation light in the testing pattern is operated.

7. A program that causes a computer to execute:
a projection control step of irradiating stimulation light with a testing pattern that increases an intensity of the stimulation light to a predetermined luminance over time; and
a calculation step of calculating the intensity of the stimulation light that is visually recognizable by a subject based on a timing at which a switch operated by the subject who visually recognizes the stimulation light in the testing pattern is operated.
